# EUROPEAN PATENT SPECIFICATION

(11) **EP 1 230 938 B1**
(45) Date of publication and mention of the grant of the patent: **20.07.2005**
(21) Application number: 00970062.6
(22) Date of filing: 25.10.2000
(51) Int. Cl.: A61L 27/32, A61F 2/08, A61L 27/38

(54) **VITAL TISSUE FOR TENDON OR LIGAMENT AND PROCESS FOR PRODUCING THE SAME**
LEBENDES GEWEBE FÜR SEHNEN UND BÄNDER UND EIN HERSTELLUNGSVERFAHREN DAFÜR
TISSU VITAL POUR TENDON ET LIGAMENT ET PROCEDE DE PRODUCTION CORRESPONDANT

(30) Priority: 15.11.1999 JP 32375399
(43) Date of publication of application: 14.08.2002
(73) Proprietor: National Institute for Materials Science, Tsukuba-shi, Ibaraki 305-0047 (JP); Taki Chemical Co., Ltd., Kakogawa-shi, Hyogo-ken 675-0124 (JP); NOF CORPORATION, Tokyo 150-0013 (JP)
(72) Inventor: TANAKA, Junzo, Nat. Inst. of Materials Science, Tsukuba-shi, Ibaraki 305-0047 (JP); KIKUCHI, Masanori, Nat. Inst. of Materials Science, Tsukuba-shi, Ibaraki 305-0047 (JP); FUKUZAKI, Hironobu, Tatsuno-shi, Hyogo 679-4156 (JP); YAMAGUCHI, Isamu 103, Mizunuki Shataku, Hyogo 675-0131 (JP); KATO, Kenji c/o NOF CORPORATION, Tokyo 150-6019 (JP); SONODA, Kensaku c/o NOF CORPORATION, Aichi 470-2345 (JP)
(74) Representative: Hallybone, Huw George
(86) International application number: PCT/JP2000/007461
(87) International publication number: WO 2001/036012

(56) References cited:
- JP-A- 6 339 521
- JP-A- 8 040 711
- JP-A- 10 127 752
- JP-A- 11 171 516

## Description

The present invention relates to a biocompatible tissue for tendons or ligaments which can be used in reconstructive surgery of tendons and ligaments and enables rapid postoperative fixation to bone and early recovery owing to its enhanced biocompatibility. The present invention also relates to a method for producing such a biocompatible tissue.

Reconstructive materials are generally used in reconstructive surgery of tendons and ligaments in order to minimize damage to healthy self-tissue and minimize the amount of self-tissue removal. Known techniques of reconstructive surgery include a technique in which nondegradable, highly strong synthetic high molecular material as a reconstructive material is used as substitutes for self-tissue, a technique in which artificial ligaments composed of self-tissue and synthetic high molecular material (*e*.*g*., polypropylene) are used to bear some of the mechanical stress that applies to the reconstructed ligaments until self-tissue is regenerated so as to enable early post-operative rehabilitation, and a technique in which biodegradable artificial ligaments are used to serve as ligaments while self-tissue is being induced.

However, all of these techniques are accompanied by a problem of the insufficient fixation rate or strength of the tendons or ligaments to bone after reconstructive surgery.

For this reason, reconstructive surgery which relies on self-transplantation rather than on use of artificial ligaments has become increasingly common. For example, a patellar tendon with a fragment of bone attached thereto or a hamstring tendon is used as self-tissue in reconstructive surgery of anterior cruciate ligament.

The purpose of using the patellar tendon with its associated bone fragment is to permit the use of interference screws by taking advantage of the innate attachments of bone/ligaments, thereby facilitating fast reconstruction. This type of reconstructive surgery provides better initial fixation and enables coaptation within a bone tunnel at an early stage. However, such reconstructive surgery is associated with problems such as relatively frequent occurrence of complications such as post-operative knee stretching impairment, knee pain and fracture of patella and necessity of skill for screw fixation.

The advantage of using hamstring tendons is that there are few complications associated with the removal of the self-tendons and that the procedure is relatively simple and has a large degree of freedom. However, this approach also has drawbacks such as weak initial fixation strength resulting in a prolonged fixation time of bone to soft tissue, and a extended rehabilitation period.

For obtaining better fixation of bone with soft tissue, i.e. tendons or ligaments, a method is proposed in which collagen and BMP (bone morphogenic protein) are used to induce generation of bone formation at an early stage in the region where the bone abuts the soft tissue. However, the method has not result in success.

It is a well-known fact that calcium phosphate compounds have excellent biocompatibility, bone inducibility, and bone conductibility and may be used as substitutes for bone and as prosthetic materials for medical use. However, techniques have yet to be established for artificially fixing fine powders of calcium phosphate compounds such as hydroxyapatite and tricalcium phosphate in soft tissue such as tendons and ligaments.

Accordingly, it is an objective of the present invention to provide a biocompatible tissue for tendons or ligaments which enables rapid postoperative fixation to bone and early recovery owing to its enhanced biocompatibility, and a method for producing such biocompatible tissue.

It is another objective of the present invention to provide a method for producing a biocompatible tissue for tendons or ligaments which enables rapid postoperative fixation to bone and early recovery owing to its enhanced biocompatibility in a simple manner and in a short period of time.

The present inventors have made extensive research and found out that, by alternately immersing a substrate for tendon or ligament tissue in a calcium solution followed by rinsing in saline and in a phosphate solution followed by rinsing in saline, the highly biocompatible calcium phosphate compound such as hydroxyapatite can be produced and firmly fixed on the surface of or within the substrate in a short period of time. The present inventors have further found out that this production method through the alternate immersion process makes it easy to control the amount of the calcium phosphate compound produced and to control the fixation of the calcium phosphate compound while maintaining physical properties of the substrate required for the tissue for tendons or ligaments, thereby completing the present invention.

According to the present invention, there is provided a biocompatible tissue for a tendon or a ligament comprising a tendon or ligament tissue substrate and a calcium phosphate compound, wherein the calcium phosphate compound is fixed at least to a surface of the tendon or ligament tissue substrate.

According to the present invention, there is also provided a method for producing the above-described biocompatible tissue for a tendon or a ligament. The method includes the step of alternately immersing the tendon or ligament tissue substrate in a calcium solution containing calcium ions but substantially no phosphate ions (which may be referred to hereinafter as a calcium solution (A)) and in a phosphate solution containing phosphate ions but substantially no calcium ions (which may be referred to hereinafter as a phosphate solution (B)), to produce and fix the calcium phosphate compound at least on the surface of the substrate.

According to the present invention, there is further provided a method for treating a tendon or a ligament including the steps of collecting from an animal including a human a self-tissue for tendons or ligaments as a substrate, alternately immersing the substrate in the calcium solution (A) and in the phosphate solution (B) to produce and fix the calcium phosphate compound at least on a surface of the substrate to obtain the above-described biocompatible tissue for the tendon or ligament, and implanting the resulting biocompatible tissue for the tendon or ligament in a defective tendon or ligament of an animal including a human.

The present invention will now be described in detail.

The biocompatible tissue of the present invention for a tendon or a ligament, which can be used as substitutes for tendons and ligaments in reconstructive surgery of tendons and ligaments in animals including humans, includes a tendon or ligament tissue substrate (which may be referred to hereinafter as substrate) and a calcium phosphate compound.

The substrate may be any substrate that can serve as a tendon or a ligament of an animal including a human. For example, the substrate composed of one or more of materials selected from the group consisting of an artificial tendon, an artificial ligament, a natural tendon of an animal, and a natural ligament of an animal may be used. The substrate using the natural tendon of an animal or the natural ligament of an animal are particularly preferred. Any known artificial tendons or ligaments may serve as the artificial tendons or ligaments of the present invention while natural tendons and ligaments surgically extracted from animals may be used as the natural tendons and ligaments of animals of the present invention. Among natural tendons and ligaments, self-tissue is particularly preferred. These materials may be subjected to various medically acceptable treatments, or they may include other materials provided that the other materials do not adversely affect the intended purpose of the invention.

In the tendon or ligament tissue of the present invention, the calcium phosphate compound to be fixed to the substrate may be any calcium phosphate compound obtained through a reaction between a phosphate and calcium. Examples thereof may include hydroxyapatite, tricalcium phosphate, octacalcium phosphate and mixtures thereof. Of these, hydroxyapatite is the most preferred since it has the highest compatibility with living bodies. While the calcium phosphate compounds in the present invention do not include natural bone, the tendon or ligament tissue of the present invention may include natural bone.

In the biocompatible tissue for the tendon or ligament of the present invention, the calcium phosphate compound is fixed at least to a surface of the substrate. It is preferred that the calcium phosphate compound be fixed also within the substrate in order to facilitate fast fixation of the tissue to bone after reconstructive surgery. The term "fixed" as used herein means that the calcium phosphate compound does not come off the substrate through the regular process of washing the substrate several times. In one preferred embodiment of the present invention which is provided by a method of the present invention described below, the calcium phosphate compound is directly fixed to the substrate without using adhesives, which may affect biocompatibility.

In the biocompatible tissue for the tendon or ligament of the present invention, the calcium phosphate compound may be fixed in an amount of 0.5 to 20wt%, in particular 1 to 10wt%, with respect to the total amount of the biocompatible tissue for the tendon or ligament in order to ensure fast fixation of the tissue to bone although the amount may not necessarily fall in the specified ranges. Further, the amount of hydroxyapatite in the calcium phosphate compound may preferably be from 50 to 100wt% so that hydroxyapatite constitutes the primary component of the calcium phosphate compound.

When necessary, the biocompatible tissue for the tendon or ligament of the present invention may be subjected to various medically acceptable treatments or they may include other materials provided that the other materials do not adversely affect the intended purpose of the invention.

The biocompatible tissue for the tendon or ligament of the present invention may be used in reconstructive surgery of tendons or ligaments. In cases where self-tissue is used as the substrate, the self-tissue to serve as tendons or ligaments may be surgically collected, and the biocompatible tissue for the tendon or ligament of the present invention may be prepared by fixing the calcium phosphate compound to the collected self-tissue during reconstructive surgery. The biocompatible tissue so prepared may then be used in the reconstructive surgery.

Thus, the present invention also provides a method for treating tendons or ligaments. The method involves collecting from an animal including a human self-tissue for a tendon or ligament to serve as the substrate, producing the calcium phosphate compound and fixing it at least to a surface of the substrate through the alternate immersion process, which is described in detail later, to obtain the biocompatible tissue for the tendon or ligament, and implanting the biocompatible tissue for the tendon or ligament in a defective tendon or ligament of an animal including a human.

In the method for producing the biocompatible tissue for the tendon or ligament of the present invention, it is preferred that the calcium phosphate compound be readily fixed to the substrate directly and in a short period of time. For example, the method may include the step of alternately immersing a substrate for the tendon or ligament tissue in a calcium solution (A) and in a phosphate solution (B) (which may be referred to hereinafter as the alternate immersion step) to produce the calcium phosphate compound and fix it at least to the surface of the substrate, but is not limited thereto.

The calcium solution (A) used in the aforementioned alternate immersion step may be an aqueous solution containing calcium ions but substantially no phosphate ions. In the presence of phosphate ions, the rate at which hydroxyapatite is produced may be decreased. For this reason, the calcium solution (A) may be formed as an aqueous solution containing calcium ions but no phosphate ions. Examples of the calcium solution may include an aqueous solution of calcium chloride, an aqueous solution of calcium acetate and buffered solutions thereof. Examples of the buffering agent may include a tris(hydroxymethyl)aminomethane-HCl buffer (referred to hereinafter as Tris buffer), but is not limited thereto.

The calcium solution (A) preferably has a calcium ion concentration of 0.1 to 40wt%, particularly 1 to 10wt%, in view of the production rate of the calcium phosphate compound and the production efficiency of the compound. The concentration of calcium ions less than 0.1wt% is not preferable since the amount of calcium phosphate compound produced in each alternate immersion step may be so small that the step may need to be repeated many times to produce calcium phosphate compound in desired amounts.

The pH of the calcium solution (A) is preferably adjusted to a value of 7 to 9 using for example a Tris buffer in order to reduce the risk of damaging living tissue though the pH is not necessarily limited to the specified range.

The phosphate solution (B) used in the alternate immersion step may be an aqueous solution containing phosphate ions but substantially no calcium ions. In the presence of calcium ions, the rate at which hydroxyapatite is produced may be decreased. For this reason, the phosphate solution (B) may be formed as an aqueous solution containing phosphate ions but no calcium ions. Examples of the phosphate solution may include an aqueous solution of sodium dihydrogenphosphate, an aqueous solution of disodium hydrogenphosphate, an aqueous solution of diammonium hydrogenphosphate, and buffered solutions thereof. Use of the aqueous solution of dihydrogensodium phosphate or a buffered solution thereof is particularly preferred in order to reduce damage to living tissue and enhance the production of hydroxyapatite. Examples of the buffering agent may include a Tris buffer, but is not limited thereto.

The phosphate solution (B) preferably has a phosphate ion concentration of 0.1 to 20wt%, particularly 0.5 to 10wt%, in view of the production rate of the calcium phosphate compound and the production efficiency of the compound. The concentration of phosphate ions less than 0.1wt% is not preferable since the amount of calcium phosphate compound produced in each alternate immersion step may be so small that the step may need to be repeated many times to produce calcium phosphate compound in desired amounts.

The pH of the phosphate solution (B) is preferably adjusted to a value of 7 to 9 using for example a Tris buffer in order to reduce the risk of damaging living tissue though the pH is not necessarily limited to the specified range.

The calcium solution (A) and the phosphate solution (B) may contain other types of ions provided that the other ions do not adversely affect the intended purpose of the invention.

The methods for immersing the substrate in the calcium solution (A) and the phosphate solution (B) in the alternate immersion step may include:
(1) performing one or more cycles of a series of operation of immersing the substrate first in the calcium solution (A) and then in the phosphate solution (B), and
(2) performing one or more cycles of a series of operation of immersing the substrate first in the phosphate solution (B) and then in the calcium solution (A).

The amount of the calcium phosphate compound produced can be increased by increasing the number of repeats of the operation. The number of repeats of the operation may generally be from 1 to 20 times. When the series of the operation (1) is repeated more than once, the final immersion is not necessarily the immersion in the phosphate solution (B) and may be in the calcium solution (A). Similarly, when the series of the operation (2) is repeated more than once, the final immersion is not necessarily the immersion in the calcium solution (A) and may be in the phosphate solution (B).

The time for which the substrate is immersed in the calcium solution (A) and the phosphate solution (B) may be properly adjusted in view of the rate and efficiency of the production of the calcium phosphate compound and the ion concentration of each solution. In general, the total immersion time is preferably selected to be several tens of seconds to one hour, in particular, 1 to 30 minutes. When the series of the operation of immersing the substrate in the calcium solution (A) and the phosphate solution (B) is repeated, the immersion time per cycle of the immersion step may be suitably selected in consideration of the preferred total immersion time.

The temperature of each solution during the immersion of the substrate in the solution is properly selected in view of the rate and the efficiency of the production of the calcium phosphate compound and may generally be selected to be a temperature of 15 to 40°C.

The alternate immersion step includes in each of the above-described cycle a step of removing the calcium solution (A) remaining on the substrate, after the substrate is immersed in the calcium solution (A) and before the substrate is immersed in the phosphate solution (B), and/or, a step of removing the phosphate solution (B) remaining on the substrate, after the substrate is immersed in the phosphate solution (B) and before the substrate is immersed in the calcium solution (A), in order to ensure firm fixation of the calcium phosphate compound and removal of excess calcium ions and phosphate ions.

The process of removing the calcium solution (A) or the phosphate solution (B) remaining on the substrate comprises rinsing with saline. While the rinsing process may only involve simply immersing the substrate in the saline, it may also involve gently stirring the saline or gently vibrating the substrate. Preferably, the temperature of the saline may be selected to be in the range of 15 to 40°C.

The production method of the present invention may include other steps so long as the method includes the above-described alternate immersion step and the biocompatible tissue for the tendon or ligament of the present invention is achieved.

While the present invention is hereinbelow described in further detail with reference to Examples, the present invention is not limited thereto. As used herein, the sign % always signifies wt% unless otherwise specified.

### Example 1

Free tendons were surgically removed from a hind leg of a sacrificed Japanese White rabbit. At room temperature, the removed free tendons were immersed for 5 minutes in an aqueous solution of calcium chloride (24°C) having a calcium ion (Ca²⁺) concentration of 0.8% and pre-conditioned to pH7.4 using a Tris buffer. Subsequently, the tendons were immersed in saline for 30 seconds and then in an aqueous solution of disodium hydrogenphosphate having a phosphate ion (PO₄²⁻) concentration of 1.1% for 5 minutes. This process was performed once or repeated 3, 6 and 8 times, and the tendons were rinsed with saline for 5 minutes to prepare biocompatible tissues for use as tendons.

A visual observation of each biocompatible tissue for the tendon revealed that the tissue turned increasingly white as the number of repeats of the alternate immersion was increased. Also, an observation of cross-sections taken along a line perpendicular to longitudinal axis of the biocompatible tissue for the tendon revealed that the color change to white also occurred within the tissue for the tissue subjected to 3 times or more of the alternate immersion.

The resulting biocompatible tissue for the tendon was freeze-dried and the amounts of samples produced, *i.e*., the amounts of the calcium phosphate compound produced, were measured using a thermo-analyzer (DTA-TG) (manufactured by RIGAKU Corporation, model No. TG8120). The results are shown in Table 1.

Further, the samples obtained by repeating the alternate immersion 8 times were baked in an electric furnace at 1200°C and were then powdered. It was determined that the resulting powder contained hydroxyapatite as its principal component by measuring the powder with a powder X-ray diffraction analyzer (XRD) (manufactured by Phillips Petroleum Company, model No. PW1729). It is thus confirmed that hydroxyapatite was the primary component of the white products.

### Example 2

Free tendons were surgically removed from a hind leg of a sacrificed Japanese White rabbit. At room temperature, the removed free tendons were immersed in an aqueous solution of calcium chloride (24°C) having a calcium ion (Ca²⁺) concentration of 4.8% for 2 minutes. Subsequently, the tendons were immersed in a saline for 30 seconds and then in an aqueous solution of disodium hydrogenphosphate having a phosphate ion (PO₄²⁻) concentration of 6.8% for 2 minutes. This process was performed once or repeated twice, 6 and 8 times, and the tendons were rinsed with saline for 5 minutes to prepare biocompatible tissues for use as tendons.

A visual observation of each biocompatible tissue for the tendon revealed that the tissue turned increasingly white as the number of repeats of the alternate immersion was increased. Also, an observation of cross-sections taken along a line perpendicular to longitudinal axis of the biocompatible tissue for the tendon revealed that the color change to white also occurred within the tissue for the tissue subjected to 6 times or more of the alternate immersion.

The resulting biocompatible tissue for the tendon was freeze-dried and the amount of the calcium phosphate compound was determined as in Example 1. The results are shown in Table 1.

**Table 1**

| | Amount of calcium phosphate produced at each immersion number (%) | | | | |
|---|---|---|---|---|---|
| | Immersion once | Immersion twice | Immersion 3 times | Immersion 6 times | Immersion 8 times |
| Example 1 | 0.9 | - | 3.7 | 6.8 | 8.5 |
| Example 2 | 1.1 | 4.2 | - | 7.4 | 9.0 |

### Example 3

Free tendons were surgically removed from a hind leg of a sacrificed Japanese White rabbit. At room temperature, the removed free tendons were immersed for 5 minutes in an aqueous solution of calcium acetate (26°C) having a calcium ion (Ca²⁺) concentration of 1.6% preconditioned to pH7.4 using a Tris buffer. Subsequently, the tendons were immersed in saline for 30 seconds and then in an aqueous solution of disodium hydrogenphosphate having a phosphate ion (PO₄²⁻) concentration of 2.3% for 5 minutes. This process was repeated 5 times and the tendons were rinsed with saline for 5 minutes to prepare a biocompatible tissue for use as a tendon. A visual observation of the biocompatible tissue for tendons revealed that the tissue turned white on its surface as well as its interior.

The resulting biocompatible tissue for the tendon was freeze-dried, and the amount of the calcium phosphate compound was measured as in Example 1 and was determined to be 7.5%.

The biocompatible tissue for the tendon or ligament of the present invention is useful in reconstructive surgery of tendons or ligaments since the calcium phosphate compound, fixed at least to a surface of the tendon or ligament tissue substrate, does not come off the substrate easily. Further, the biocompatible tissue for tendon or ligament of the present invention enables fast induction of bone tissue formation as well as firm fixation of the tissue to bone after surgery based on the biocompatibility of the fixed calcium phosphate compound.

The production method of the present invention, which involves the alternate immersion process, makes it possible for the calcium phosphate compound to be produced and fixed directly to the substrate. Furthermore, since the method of the present invention may be performed with the simple steps of the alternate immersion, the method makes it easy to control the fixation of the calcium phosphate compound while maintaining physical properties of the substrate required for the tissue for tendons or ligaments. It also enables the production and fixation of the calcium phosphate compound in a short period of time. Thus, the ligament or tendon tissue of the present invention can be prepared while reconstructive surgery is being performed.

## Claims

1. A method for producing a biocompatible tissue for a tendon or a ligament comprising a tendon or ligament tissue substrate and a calcium phosphate compound, the calcium phosphate compound being fixed at least to a surface of the tendon or ligament tissue substrate, the method comprising the step of;
alternately immersing the tendon or ligament tissue substrate in a calcium solution containing calcium ions but substantially no phosphate ions and in a phosphate solution containing phosphate ions but substantially no calcium ions, to produce and fix the calcium phosphate compound at least on the surface of the substrate,
wherein the calcium solution remaining on the tendon or ligament tissue substrate is removed by rinsing the substrate with saline after the substrate is immersed in the calcium solution and before the substrate is immersed in the phosphate solution, and the phosphate solution remaining on the tendon or ligament tissue substrate is removed by rinsing the substrate with saline after the substrate is immersed in the phosphate solution and before the substrate is immersed in the calcium solution.

2. The method according to claim 1, wherein the tendon or ligament tissue substrate is a substrate composed of one or more of materials selected from the group consisting of an artificial tendon, an artificial ligament, a natural tendon of an animal, and a natural ligament of an animal

3. The method according to claim 1, wherein the calcium solution containing calcium ions but substantially no phosphate ions has a calcium ion concentration of 0.1 to 40wt% and the phosphate solution containing phosphate ions but substantially no calcium ions has a phosphate ion concentration of 0.1 to 20wt%.

4. The method according to claim 1, wherein a condition in which the tendon or ligament tissue substrate is alternately immersed in the calcium solution and in the phosphate solution is adjusted so that the calcium phosphate compound produced and fixed includes hydroxyapatite.

## Patentansprüche

1. Verfahren zur Herstellung eines biokompatiblen Gewebes für eine Sehne oder ein Ligament, das ein Sehnen- oder Ligamentgewebesubstrat und eine Calciumphosphatverbindung umfasst,
wobei die Calciumphosphatverbindung zumindest an einer Oberfläche des Sehnen- oder Ligamentgewebesubstrats fixiert ist,
wobei das Verfahren den folgenden Schritt umfasst:
Abwechselndes Eintauchen des Sehnen- oder Ligamentgewebesubstrats in eine Calciumlösung, die Calcium-Ionen, aber im Wesentlichen keine Phosphat-Ionen enthält, und in eine Phosphatlösung, die Phosphat-Ionen, aber im Wesentlichen keine Calcium-Ionen enthält, um die Calciumphosphatverbindung zumindest an der Oberfläche des Substrats herzustellen und zu fixieren,
wobei die Calciumlösung, die auf dem Sehnen- oder Ligamentgewebesubstrat zurückbleibt, durch Spülen des Substrats mit Kochsalzlösung, nachdem das Substrat in die Calciumlösung eingetaucht wurde und bevor das Substrat in die Phosphatlösung eingetaucht wird, entfernt wird und die Phosphatlösung, die auf dem Sehnen- oder Ligamentgewebesubstrat zurückbleibt, durch Spülen des Substrats mit Kochsalzlösung, nachdem das Substrat in die Phosphatlösung eingetaucht wurde und bevor das Substrat in die Calciumlösung eingetaucht wird, entfernt wird.

2. Verfahren nach Anspruch 1, wobei das Sehnen- oder Ligamentgewebesubstrat ein Substrat ist, das aus einem Material oder mehreren Materialien, ausgewählt aus der Gruppe bestehend aus einer künstlichen Sehne, einem künstlichen Ligament, einer natürlichen Sehne eines Tiers und einem natürlichen Ligament eines Tiers, besteht.

3. Verfahren nach Anspruch 1, wobei die Calciumlösung, die Calcium-Ionen, aber im Wesentlichen keine Phosphat-Ionen enthält, eine Calcium-Ionenkonzentration von 0,1-40 Gew.-% hat und die Phosphatlösung, die Phosphat-Ionen, aber im Wesentlichen keine Calcium-Ionen enthält, eine Phosphat-Ionenkonzentration von 0,1-20 Gew.-% hat.

4. Verfahren nach Anspruch 1, wobei die Bedingungen, unter denen das Sehnen- oder Ligamentgewebesubstrat abwechselnd in die Calciumlösung und in die Phosphatlösung eingetaucht wird, so eingestellt werden, dass die Calciumphosphatverbindung, die produziert und fixiert wird, Hydroxyapatit umfasst.

## Revendications

1. Méthode pour la production d'un tissu biocompatible pour un tendon ou un ligament comprenant un substrat de tissu pour tendon ou pour ligament et un composé phosphate de calcium, le composé phosphate de calcium étant fixé au moins à une surface du substrat de tissu pour tendon ou pour ligament, la méthode comprenant l'étape de :
l'immersion alternative du substrat de tissu pour tendon ou pour ligament dans une solution de calcium contenant des ions calcium, mais ne contenant substantiellement pas d'ions phosphate et dans une solution de phosphate contenant des ions phosphates, mais ne contenant substantiellement pas d'ions calcium, pour produire et fixer le composé phosphate de calcium au moins sur la surface du substrat,
la solution de calcium restant sur le substrat de tissu pour tendon ou pour ligament étant enlevée par rinçage du substrat avec une solution saline après immersion du substrat dans la solution de calcium et avant immersion du substrat dans la solution de phosphate, et la solution de phosphate restant sur le substrat de tissu pour tendon ou pour ligament étant enlevée par rinçage du substrat avec une solution saline après immersion du substrat dans la solution de phosphate et avant immersion du substrat dans la solution de calcium.

2. Méthode selon la revendication 1, dans laquelle le substrat de tissu pour tendon ou pour ligament est un substrat composé d'une ou plusieurs matières choisies dans le groupe constitué par un tendon artificiel, un ligament artificiel, un tendon naturel d'un animal et un ligament naturel d'un animal.

3. Méthode selon la revendication 1, dans laquelle la solution de calcium contenant des ions calcium, mais ne contenant substantiellement pas d'ions phosphate a une concentration en ion calcium de 0,1 à 40 % en masse et la solution de phosphate contenant des ions phosphates, mais ne contenant substantiellement pas d'ions calcium, a une concentration en ion phosphate de 0,1 à 20 % en masse.

4. Méthode selon la revendication 1, dans laquelle une condition de l'immersion du substrat de tissu pour tendon ou pour ligament alternativement dans la solution de calcium et dans la solution de phosphate est ajustée de telle façon que le composé phosphate de calcium produit et fixé inclut de l'hydroxyapatite.
